(19) Europäisches Patentamt
European Patent Office
Office européen des brevets

(11) Numéro de publication : **0 226 519**
**B1**

(12) # FASCICULE DE BREVET EUROPÉEN

(45) Date de publication du fascicule du brevet : **24.01.90**

(51) Int. Cl.$^5$ : **C 07 C 59/01**, C 07 C 51/10

(21) Numéro de dépôt : **86420284.1**

(22) Date de dépôt : **21.11.86**

(54) **Procédé de préparation d'alpha-hydroxyacides.**

(30) Priorité : **27.11.85 FR 8517748**

(43) Date de publication de la demande :
**24.06.87 Bulletin 87/26**

(45) Mention de la délivrance du brevet :
**24.01.90 Bulletin 90/04**

(84) Etats contractants désignés :
**AT BE CH DE ES FR GB GR IT LI LU NL SE**

(56) Documents cités :
**Néant**

(73) Titulaire : **RHONE-POULENC CHIMIE**
**25, quai Paul Doumer**
**F-92408 Courbevoie Cédex (FR)**

(72) Inventeur : **Besson, Bernard**
**1, rue du Tonkin**
**F-69100 Villeurbanne (FR)**

(74) Mandataire : **Le Pennec, Magali et al**
**RHONE-POULENC INTERSERVICES** Service Brevets
**Chimie 25, Quai Paul Doumer**
**F-92408 Courbevoie Cédex (FR)**

**EP 0 226 519 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé de préparation d'alpha-hydroxyacides carboxyliques par double carbonylation réductrice d'halogénures d'alkyles.

Les acides alpha-hydroxycarboxyliques tels que l'acide lactique, l'acide alpha-hydroxybutyrique, l'acide alpha-hydroxy-octanoïque, sont des produits importants du point de vue industriel. Ils sont utilisés soit directement (par exemple dans le domaine alimentaire pour l'acide lactique), soit comme intermédiaire en synthèse organique. Il existe diverses voies d'accès aux alpha-hydroxyacides. Les plus intéressantes au plan industriel sont la fermentation (préparation des acides lactique et alpha-hydroxybutyrique notamment), l'hydrolyse en milieu alcalin des acides alpha-halogénocarboxyliques, l'hydrolyse acide des cyanohydrines d'aldéhydes (alpha-hydroxynitriles). Quel que soit l'intérêt de ces divers procédés, ils souffrent d'inconvénients qui en limite l'application. Ainsi les procédés de fermentation ne sont pas d'utilisation générale et conduisent à des mélanges complexes à partir desquels la récupération de l'alpha-hydroxyacide recherché s'avère compliquée. Les procédés de synthèse organique impliquent la préparation de produits de départ par la mise en œuvre de méthodes multiétapes (préparation des alpha-halogénoacides ou des alpha-hydroxynitriles par exemple). Pour toutes ces raisons l'industrie est à la recherche de procédés de préparation d'alpha-hydroxyacides mettant en œuvre des réactifs facilement accessibles. La présente invention a précisément pour objet un tel procédé.

Plus spécifiquement la présente invention a pour objet un procédé de préparation d'acides alpha-hydroxycarboxyliques, caractérisé en ce que l'on fait réagir un halogénure d'alkyle avec de l'oxyde de carbone et un composé donneur d'hydrogène, en présence d'un catalyseur de carbonylation et d'une base minérale, à une température supérieure ou égale à 90 °C.

Au titre de la présente invention, le terme « halogénure d'alkyle » désigne, par raison de commodité, tout composé comportant un reste de formule :

$$—CH_2—X$$

(dans laquelle X représente un atome d'halogène), lié soit à un reste alkyle linéaire ou ramifié, substitué ou non par des restes fonctionnels ou des radicaux cycloalkyles, aryles, aryloxy ou hétérocycliques, soit à un reste aryloxy.

Les termes « radical alkyle inférieur et alkoxy inférieur » désigneront par la suite des radicaux alkyles ou alkyloxy, linéaires ou ramifiés, comportant de 1 à 4 atomes de carbone.

L'obtention d'alpha-hydroxyacides par réaction d'oxyde de carbone avec un halogénure d'alkyle et un composé donneur d'hydrogène, en présence d'un catalyseur de carbonylation, à une température supérieure à 90 °C est un résultat totalement inattendu au regard de l'enseignement de l'état de la technique. En effet, dans la demande internationale publiée sous le No. WO 84/02699 on a décrit un procédé de préparation d'acides alpha-céto-carboxyliques par double carbonylation d'un halogénure organique pris dans le groupe formé par les halogénures d'alkyle, de phénoxyalkyle ou de phénylalkyle dans lesquels le reste phényle est au moins en position alpha par rapport à l'atome de carbone portant l'atome d'halogène dans un milieu hydroorganique et en présence d'un catalyseur de carbonylation. Cette demande internationale enseigne que les acides alpha-céto-carboxyliques sont obtenus majoritairement quelles que soient les conditions mises en œuvre et en particulier quels que soient les solvants utilisés (alcools primaires, secondaires ou tertiaires) et la température mise en œuvre (de 30 à 150 °C). Il s'avère donc surprenant pour l'homme de métier qu'en conduisant la réaction d'un halogénure d'alkyle à une température supérieure à 90 °C et en présence d'un composé donneur d'hydrogène, il se forme majoritairement un alpha-hydroxyacide au lieu d'un alpha-cétoacide.

Plus spécifiquement la présente invention a pour objet un procédé de préparation d'acides alpha-hydroxycarboxyliques de formule générale :

$$\begin{array}{c} R - \underset{\displaystyle R_1}{\underset{|}{CH}} - \underset{\displaystyle OH}{\underset{|}{CH}} - COOH \end{array} \qquad (I)$$

dans laquelle R et $R_1$, identiques ou différents, représentent :

un atome d'hydrogène ;

un radical alkyle, linéaire ou ramifié, comportant de 1 à 20 atomes de carbone, éventuellement substitué par :

a) un ou plusieurs restes fonctionnels inertes dans les conditions de la réaction tels que nitro, nitrile, alkylcarbonyloxy ;

b) un ou plusieurs restes alkyles ;

c) un ou plusieurs restes cycloalkyles ;

d) un ou plusieurs restes hétérocycliques comportant un ou plusieurs hétéroatomes pris dans le

phénoxy-4 butyle.

Comme composé donneur d'hydrogène convenant à la formation d'un alpha-hydroxyacide on peut faire appel à tout composé organique comportant des atomes d'hydrogène labiles dans les conditions de la réaction, c'est-à-dire en présence d'oxyde de carbone, d'une base minérale et d'un catalyseur de carbonylation et à chaud. Une classe préférée de donneurs d'hydrogène est constituée par les alcools primaires et secondaires aliphatiques saturés. Plus spécifiquement on fait appel à des alcools de formule générale :

$$R_2—CHOH—R_3 \qquad (VI)$$

dans laquelle $R_2$ représente un radical hydrocarboné comportant de 1 à 20 atomes de carbone et $R_3$ un atome d'hydrogène ou un reste hydrocarboné identique ou différent de $R_2$. Dans la formule (VI) $R_2$ et/ou $R_3$ représentent plus particulièrement des radicaux alkyle, arylalkyle, aryle, alkylaryle ou cycloalkyle ; comme exemple de radicaux $R_2$ et $R_3$, on peut citer les radicaux méthyle, éthyle, n-propyle, sec-butyle, cyclohexyle, phényle, benzyle, xylyles, toluyles. Des alcools comme le méthanol, l'éthanol, le propanol-1, l'isopropanol, l'isobutanol, le diméthyl-2,2 propanol-1, le butanol-2, le méthyl-3 butanol-1, l'isopentanol peuvent être cités à titre d'exemples non limitatifs. Les alcools secondaires sont utilisés préférentiellement.

Sans que l'invention soit limitée en quoi que ce soit à un mécanisme réactionnel particulier elle peut être représentée par le schéma réactionnel suivant lorsque le donneur d'hydrogène est un alcool :

$$(1)\ R-\underset{\underset{R_1}{|}}{CH}-X + R_2-\underset{\underset{R_3}{|}}{CHOH} + CO + 2\,OH^- \longrightarrow R-\underset{\underset{R_1}{|}}{CH}-CHOH-COO^- + H_2O + R_2-CO-R_3$$

dans lequel R, $R_1$, $R_2$, $R_3$ et X ont la signification donnée auparavant.

La quantité de donneur d'hydrogène exprimée en mole par mole d'halogénure d'alkyle peut varier dans de larges limites en fonction de la nature du donneur. Cette quantité est de préférence au moins égale à 1 mole par mole d'halogénure d'alkyle et plus préférentiellement encore au moins égale à 5 moles par mole. Il n'y a pas de limite supérieure critique de la quantité de donneur d'hydrogène et on peut mettre en œuvre un large excès de ce dernier ; lorsqu'il est liquide dans les conditions de la réaction il peut être utilisé avantageusement comme milieu de réaction. On pourrait sans sortir du cadre de la présente invention mettre en œuvre moins de 1 mole de donneur d'hydrogène par mole d'halogénure, toutefois une quantité insuffisante de donneur se traduirait par une transformation incomplète de l'halogénure.

La base minérale utilisée pour conduire la réaction est choisie parmi les hydroxydes alcalins (tels que la soude, la potasse ou l'hydroxyde de lithium) ou les hydroxydes alcalino-terreux tels que $Ba(OH)_2$, $Ca(OH)_2$ ou $Mg(OH)_2$, ou les carbonates alcalins ou alcalino-terreux. On a de préférence recours à LiOH ou à $Ca(OH)_2$.

La quantité de base peut varier dans de larges limites. En général on met en œuvre une quantité de base voisine de 1 mole par mole d'halogénure d'alkyle. On peut toutefois, sans sortir du cadre de la présente invention, opérer avec un excès de base qui peut atteindre une mole ou plus.

La réaction est de préférence conduite en présence d'une quantité plus importante de base qui peut être comprise entre 1,1 et 4 moles par mole d'halogénure.

Comme catalyseur on peut utiliser tout composé métallique connu pour initier les réactions de carbonylation ou de double carbonylation tels que ceux cités dans le brevet français 75/00533 ; la demande de brevet français publiée sous le No. 2 429 772 ; la demande de brevet internationale publiée sous le numéro WO 84/02699. On fait appel de préférence à des dérivés de métaux carbonylés et en particulier aux dérivés du fer, du nickel et du cobalt. On peut faire appel à des métaux carbonyles tels que le ferpentacarbonyle, le nickeltétracarbonyle, le dicobaltoctacarbonyle, le tétracobaltdodécacarbonyle, à des sels alcalins ou alcalino-terreux d'hydrures de métaux carbonyles et plus préférentiellement aux tétracarbonylcobaltates(-1) alcalins ou alcalino-terreux. On pourrait également, sans sortir du cadre de la présente invention, faire appel à des complexes carbonylés du fer, du nickel et du cobalt avec des ligands mono- ou polydentates. Les dérivés carbonylés du cobalt constituent une classe particulièrement préférée de catalyseurs.

La quantité de catalyseur exprimée en atomes-grammes de métal par mole d'halogénure d'alkyle peut être comprise entre 0,0001 et 1. De préférence cette quantité est choisie de façon à ce que le rapport précédent soit compris entre 0,001 et 0,4.

Bien que le donneur d'hydrogène puisse être utilisé comme milieu de réaction, comme cela a été indiqué auparavant, il peut se révéler avantageux d'opérer en présence d'un tiers solvant inerte dans les conditions de la réaction. Dans ce cas on a recours de préférence à l'eau ou à un alcool tertiaire tel que le t-butanol, l'alcool t-amylique, le méthyl-2 pentanol-2, le diméthyl-2,3 butanol-2 ou à un éther comme le dioxane, le tétrahydrofuranne, le diméthoxy-1,2 éthane, le diglyme. On peut, sans sortir du cadre de la

groupe formé par l'oxygène, l'azote et le soufre ;

e) un radical aryloxy.

Plus spécifiquement dans la formule (I), R et/ou $R_1$ représentent :

un radical alkyle tel que méthyle, éthyle, n-propyle, isopropyle, n-butyle, isobutyle, sec-butyle, t-butyle, n-pentyle, isopentyle, n-hexyle, n-heptyle, n-octyle, éthyl-2 hexyle, n-dodécyle, n-hexadécyle, n-nonadécyle ;

un reste arylalkyle comportant de 1 à 20 atomes de carbone dans sa partie alkyle et 1 ou 2 cycles benzéniques condensés ou non dans sa partie aromatique, lesdits cycles pouvant être substitués par un ou plusieurs radicaux alkyles linéaires ou ramifiés comportant de 1 à 4 atomes de carbone, radicaux alkoxy comportant de 1 à 4 atomes de carbone, un ou plusieurs atomes d'halogène (chlore, brome, fluor), un ou plusieurs groupes nitrile, nitro, alkylcarbonyloxy. Dans ce cas R et/ou $R_1$ représente de préférence un radical arylalkyle de formule générale :

$$(R')_n—Ar—(CH_2)—_m \qquad (II)$$

dans laquelle : Ar représente un radical aryle comportant un ou deux cycles benzéniques condensés ou non ; —R' représente un radical alkyle comportant de 1 à 4 atomes de carbone, un radical alkoxy comportant de 1 à 4 atomes de carbone, un atome d'halogène, un groupe nitrile, nitro, alkylcarbonyloxy ; n représente un nombre entier de 0 à 3 quand Ar comporte un seul cycle benzénique ou de 0 à 5 quand Ar comporte deux cycles benzéniques ; m représente un nombre entier de 1 à 20 et de préférence de 1 à 10.

Un reste aryloxyalkyle comportant de 1 à 20 atomes de carbone dans sa partie alkyle et 1 ou 2 cycles benzéniques condensés ou non dans sa partie aromatique, lesdits cycles pouvant être substitués par un ou plusieurs radicaux alkyles linéaires ou ramifiés comportant de 1 à 4 atomes de carbone, radicaux alkoxy comportant de 1 à 4 atomes de carbone, un ou plusieurs atomes d'halogène (chlore, brome, fluor), un ou plusieurs groupes nitrile, nitro, alkylcarbonyloxy. Dans ce cas R et/ou $R_1$ représente de préférence un radical arylalkyle de formule générale :

$$(R')_n—Ar—O—(CH_2)—_m \qquad (III)$$

dans laquelle R', Ar, n et m ont la définition donnée ci-avant.

Comme exemples spécifiques de radicaux arylalkyle et de radicaux aryloxyalkyle on peut citer les radicaux benzyle, phényl-1 éthyle, phényl-2 éthyle, phényl-3 propyle, phényl-2 propyle, phényl-4 butyle, ortho-toluylméthyle, xylylméthyle, ortho-méthoxyphénylméthyle, p-chlorophénylméthyle, naphtyl-2 méthyle, phényloxyméthyle, phényloxy-2 éthyle, phénoxy-2 propyle.

Enfin R et/ou $R_1$ peuvent encore représenter plus spécifiquement un reste aryloxy comportant un ou deux cycles benzéniques condensés ou non de formule générale :

$$(R')_n—Ar—O— \qquad (IV)$$

dans laquelle R', n et Ar ont la signification donnée précédemment. Comme exemples de radicaux aryloxy on peut citer les radicaux phénoxy ; o-méthylphénoxy- ; o-méthoxyphénoxy ; diméthyl-2,4 phénoxy ; naphtyloxy.

Dans la formule (I), $R_1$ représente de préférence un atome d'hydrogène ou un reste alkyle inférieur.

Comme exemples d'alpha-hydroxyacides pouvant être préparés par le procédé selon la présente invention, on citera plus particulièrement l'acide lactique ; l'acide alpha-hydroxybutyrique ; l'acide hydroxy-2 méthyl-4 pentanoïque ; l'acide alpha-hydroxypentanoïque ; l'acide hydroxy-2 diméthyl-3,3 butanoïque ; l'acide alpha-hydroxyoctanoïque ; l'acide hydroxy-2 décanoïque ; l'acide hydroxy-2 palmitique ; l'acide hydroxy-2 stéarique ; l'acide phényl-4 hydroxy-2 butanoïque.

Les halogénures d'alkyle utilisés comme produits de départ dans le procédé selon la présente invention peuvent être représentés par la formule générale :

$$\begin{array}{c} R - CH - X \\ | \\ R_1 \end{array} \qquad (V)$$

dans laquelle R et $R_1$ ont la signification donnée ci-avant et X représente un atome d'halogène et de préférence un atome de chlore ou de brome. Comme exemples d'halogénures d'alkyle de formule (V), on peut citer : le chlorure et le bromure de méthyle, le chloroéthane, le chloro-1 propane, le chloro-2 propane, le bromo-2 propane, le chloro-1 butane, le chloro-2 butane, le chlorure d'isobutyle, le bromure d'isobutyle, le chloro-1 pentane, le bromo-1 hexane, le bromo-1 octane, le chloro-1 octadécane, le chlorure et le bromure de phénéthyle, le chlorure d'ortho-méthylphényl-2 éthyle, le bromure de diméthyl-2',4' phényl-2 éthyle, le chlorure de phényl-3 propyle, le bromure de phényl-4 butyle, le chlorure de phénoxyméthyle, le bromure de phénoxy-2 éthyle, le chlorure de phénoxy-3 propyle, le chlorure de

présente invention faire appel à un mélange de deux ou plus de deux solvants inertes ; de ce point de vue l'emploi de mélanges eau/solvants organiques miscibles à l'eau convient tout particulièrement bien. Ainsi on peut faire appel à des mélanges eau/t-butanol, eau/dioxane, eau/tétrahydrofuranne.

La concentration de l'halogénure d'alkyle dans le milieu réactionnel n'est pas critique. Il est préférable qu'elle soit suffisante pour assurer une productivité aussi élevée que possible de l'appareillage.

La pression d'oxyde de carbone à laquelle on conduit la réaction peut varier dans de larges limites. En général elle peut prendre toute valeur dans un intervalle de 1 à 200 bar et de préférence de 5 à 150 bar.

Pour orienter la réaction vers la formation prépondérante d'alpha-hydroxyacide, la température mise en œuvre doit être au moins égale à 90 °C. On a constaté qu'au-delà de cette température le rendement en alpha-hydroxyacide augmente avec la température jusqu'à une valeur optimale de cette dernière qui dépend de la nature du substrat. Au-dessus de cette valeur optimale les rendements en alpha-hydroxyacides diminuent lorsque la température augmente. En général il est préférable d'opérer à température inférieure à 160 °C. Une valeur de la température prise dans un intervalle de 90 à 130 °C convient tout particulièrement bien.

Sur un plan pratique, le procédé selon l'invention est particulièrement simple à mettre en œuvre. Il suffit en effet de charger dans un appareil de réaction l'halogénure d'alkyle, le donneur d'hydrogène, éventuellement de l'eau et/ou un solvant organique inerte, le catalyseur et la base, d'introduire une quantité suffisante d'oxyde de carbone à la pression convenable puis de porter l'ensemble à la température choisie sous agitation. En fin de réaction l'alpha-hydroxyacide est présent sous forme de son sel alcalin ou alcalino-terreux et peut être récupéré par les moyens usuels. Lorsque le sel obtenu est en solution dans le milieu réactionnel on peut le précipiter par tout moyen approprié puis le séparer par filtration.

Lorsque le sel formé est insoluble ou partiellement insoluble on le filtre. Dans tous les cas l'alpha-hydroxyacide peut être libéré par acidification à l'aide d'une solution aqueuse d'un acide minéral puis récupéré par extraction par un solvant.

Le procédé selon l'invention se prête tout particulièrement bien à une mise en œuvre continue.

Les exemples suivants illustrent l'invention et montrent comment elle peut être mise en pratique.

### Exemple 1

Dans un autoclave en acier inoxydable de 125 ml préalablement purgé à l'argon on introduit :

bromo-1 octane : 79 mMol (15,3 g)
chaux : 123 mMol (9,1 g)
eau : 11,7 g
isopropanol : 46 g
$Co_2(CO)_8$ : 1,15 mMol (393 mg).

L'autoclave est fermé hermétiquement et placé dans un four agité par secousses et raccordé à un système d'alimentation de gaz sous pression. On admet à froid une pression de 20 bar de CO puis on chauffe l'autoclave jusqu'à une température de 120 °C. Lorsque cette température est atteinte, on régule la pression à 90 bar. Au bout de 2 h 50 mn, on refroidit l'autoclave et on relâche la pression. Après ouverture de l'autoclave, la masse réactionnelle est filtrée. On obtient d'une part un produit blanc solide (13,1 g) et une solution hydroalcoolique. Dans le produit solide on met en évidence de la chaux (2,4 g), 0,4 g du sel de calcium de l'acide nonanoïque, 10,2 g de sel de calcium de l'acide alpha-hydroxydécanoïque. Le rendement en acide par rapport au bromo-1 octane chargé en alpha-hydroxydécanoïque (sous forme de sel de Ca) est de 65 % (rendement réel ou RR)

L'identification des différents produits formés au cours de la réaction et présents dans les différentes phases de la masse réactionnelle est effectuée par chromatographie en phase vapeur ou par chromatographie en phase liquide sous haute pression suivant le cas par comparaison avec des échantillons dûment caractérisés. Dans le produit solide, on met en évidence de la chaux, le sel de calcium de l'acide nonaoïque et le sel de calcium de l'acide alpha-hydroxydécanoïque. On observe le sel de calcium de l'acide alpha-cétodécanoïque ni dans le solide, ni dans la solution hydroalcoolique.

### Exemple comparatif A

On opère de la même manière et avec les mêmes charges en poids que dans l'exemple 1 sauf que la température est de 70 °C et la durée de la réaction 16 heures.

Dans le produit solide (12,6 g) on observe uniquement la chaux, l'acide nonanoïque et l'acide alpha-cétodécanoïque sous forme de sel de Ca mais pas d'acide alpha-hydroxydécanoïque. L'acide alpha-cétodécanoïque est obtenu avec un rendement de 35 %.

### Exemple comparatif B

On opère de la même manière et avec les mêmes charges en poids que dans l'exemple 1 sauf que l'isopropanol est remplacé par le tertiobutanol.

Dans le produit solide on observe uniquement la chaux, l'acide nonanoïque et l'acide alpha-cétodécanoïque sous forme de sel de Ca mais pas d'acide alpha-hydroxydécanoïque. L'acide alpha-cétodécanoïque est obtenu avec un rendement de 56 %.

Exemple 2

On opère de la même manière et avec les mêmes charges que dans l'exemple 1 sauf que le bromo-1 octane est remplacé par le bromo-1 méthyl-2 propane dans les mêmes quantités molaires, que la pression n'est que de 60 bar et que la durée est de 18 heures.

Dans le solide (11,1 g) on dose uniquement la chaux et le sel de Ca de l'acide méthyl-4 hydroxy-2 pentanoïque : 9,4 g. On n'observe l'alpha-cétoacide correspondant qu'à l'état de traces. Le rendement en acide hydroxy-2 méthyl-4 pentanoïque est de 80 %. On dose en outre dans les eaux-mères 6 mMol d'acide méthyl-3 butanoïque sous forme de son sel de calcium.

Essai comparatif C

On opère comme dans l'exemple 2, sauf que la température est de 70 °C et la durée de réaction 18 heures. Dans le solide on n'observe pas d'alpha-hydroxyacide mais seulement l'alpha-cétoacide correspondant sous forme de sel de Ca avec un rendement de 10 %.

Essai comparatif D

On opère comme dans l'exemple 2 sauf que l'on remplace l'isopropanol par le tertiobutanol, que la durée de réaction est de 6 heures et que la pression est de 90 bar. Dans le solide on dose de l'alpha-hydroxyacide (rendement 3 %) du cétoacide (rendement 35 %) et l'acide de monocarbonylation (rendement 3 %) sous forme de leur sel de Ca.

Exemple 3

On opère comme dans l'exemple 1, sauf que le substrat engagé est le bromure de phénéthyle, la pression est de 60 bar et la durée de la réaction est de 18 heures 30 minutes. Les réactifs sont engagés dans le même rapport molaire que dans l'exemple 1. Après filtration de la masse réactionnelle on récupère 10,8 g de solide dans lequel on dose après analyse 3,5 g de chaux et 37 mMol d'acide méthyl-4 hydroxy-2 pentanoïque sous forme de sel de calcium. On n'observe pas d'acide méthyl-4 oxo-2 pentanoïque sous forme de sel de calcium ni dans les eaux-mères, ni dans le solide. Le rendement en alpha-hydroxyacide est de 47 %.

Exemple 4

On opère comme dans l'exemple 3, à ceci près que la température n'est que de 110 °C et la durée de réaction de 5 heures 30 minutes. Le substrat est le bromure de phénéthyle. Les réactifs sont engagés dans les mêmes rapports molaires. Après arrêt de la réaction, l'autoclave est refroidi puis dégazé ; la masse réactionnelle est filtrée et le produit solide obtenu rincé à l'eau. On obtient 197,5 g de solution hydroalcoolique et 15,6 g de produit solide. L'analyse de la phase hydroalcoolique montre que tout le bromure de phénéthyle a été consommé. La solution hydroalcoolique est alors concentrée à sec ; on obtient 6,2 g d'extrait sec qui est broyé avec le produit solide. Le solide ainsi obtenu est analysé par potentiométrie et chromatographie en phase liquide sous haute pression. On obtient la composition suivante : $Ca(OH)_2$ : 40,9 mMole (3,03 g)-$CaBr_2$ : 42 mMole (4,20 g), sel de calcium de l'acide phényl-4 oxo-2 butanoïque : 2,6 meq acides (0,51 g), sel de calcium de l'acide phényl-4 hydroxy-2 butanoïque : 66,6 meq acide (13,25 g), sel de calcium de l'acide phényl-3 propanoïque : 4,9 meq acide (0,8 g).

Le rendement en sel de calcium de l'acide phényl-4 hydroxy-2 butanoïque est de 84 % par rapport au bromure de phénéthyle.

Exemple 5

On opère de la même manière que dans l'exemple 3, sauf que la température de la réaction est de 155 °C et que la durée de la réaction est de 4 heures. Après filtration de la masse réactionnelle on récupère 9,9 g de produit solide et un filtrat de 46,9 g. Dans le produit solide on dose exclusivement 9 mMol d'acide méthyl-4 hydroxy-2 pentanoïque sous forme de sel de calcium. Dans la solution hydroalcoolique on dose 18 mMol d'acide méthyl-4 hydroxy-2 pentanoïque à l'exclusion d'autres produits de carbonylation.

Exemple 6

On opère de la même manière que dans l'exemple 3, sauf que la température de la réaction est de 90 °C et la durée de 19 heures 15 minutes. Après filtration de la masse réactionnelle on récupère 8,9 g de

produit solide et 64,2 g de solution hydroalcoolique. Dans le produit solide, on dose 0,2 mMol d'acide méthyl-4 oxo-2 pentanoïque, 16,6 mMol d'acide méthyl-4 hydroxy-2 pentanoïque et 1,5 mMol d'acide méthyl-3 butanoïque sous forme de leur sel de calcium. Dans la solution hydroalcoolique on dose 8,8 mMol d'acide méthyl-3 butanoïque.

Exemple 7

On opère de la même manière que dans l'exemple 3, excepté l'isopropanol est remplacé par un mélange de tertiobutanol et d'éthanol respectivement dans les quantités de 36,7 g et 9,2 g. La durée de la réaction est de 6 heures 20 minutes, les autres conditions sont inchangées. Après filtration de la masse réactionnelle, on récupère 41,7 g de solution hydroalcoolique et 6,40 g de produits solides. Les analyses montrent que dans le solide il y a 1,5 mMol de l'acide méthyl-4 oxo-2 pentanoïque et 12,8 mMol de l'acide méthyl-4 hydroxy-2 pentanoïque sous forme de sel de calcium. Dans la solution hydroalcoolique on dose uniquement 10 mMol de l'acide méthyl-4 hydroxy-2 pentanoïque sous forme de sel de calcium.

Exemple 8

On opère de la même manière, avec le même substrat et avec les mêmes charges que dans l'exemple 2, sauf que la pression est de 40 bar et la durée de la réaction est de 14 heures 30 minutes. Après refroidissement de l'autoclave et dégazage la masse réactionnelle est filtrée. Le dosage de la solution hydroalcoolique (filtrat) montre que le substrat a été totalement transformé. Le filtrat est concentré à sec ; on obtient 16,8 g de résidu. Ce résidu est mélangé avec le solide obtenu par filtration (5,75 g) et homogénéisé. Le solide ainsi obtenu est analysé. On dose les produits suivants :

$Ca(OH)_2$ : 3,41 g

acide méthyl-4 hydroxy-2 pentanoïque (sel de Ca) : 69,8 mMol (10,5 g)

acide isovalérique (sel de Ca) : 9,1 mMol (1,0 g).

Le rendement en acide méthyl-4 hydroxy-2 pentanoïque sous forme de sel de calcium est de 88,4 %.

## Revendications

1. Procédé de préparation d'acides alpha-hydroxycarboxyliques, caractérisé en ce que l'on fait réagir un halogénure d'alkyle avec de l'oxyde de carbone et un composé donneur d'hydrogène, en présence d'un catalyseur de carbonylation et d'une base minérale, à une température supérieure ou égale à 90 °C.

2. Procédé selon la revendication 1, caractérisé en ce que l'on prépare des acides alpha-hydroxycarboxyliques de formule générale :

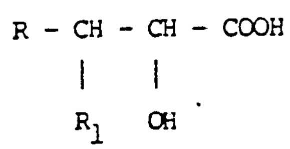

(I)

dans laquelle R et $R_1$, identiques ou différents, représentent :

un atome d'hydrogène ;

un radical alkyle, linéaire ou ramidié, comportant de 1 à 20 atomes de carbone, éventuellement substitué par :

a) un ou plusieurs restes fonctionnels inertes dans les conditions de la réaction tels que nitro, nitrile, alkylcarbonyloxy ;

b) un ou plusieurs restes aryles ;

c) un ou plusieurs restes cycloalkyles ;

d) un ou plusieurs restes hétérocycliques comportant un ou plusieurs hétéroatomes pris dans le groupe formé par l'oxygène, l'azote et le soufre ;

e) un radical aryloxy.

un radical aryloxy.

à partir d'halogénures organiques de formule générale :

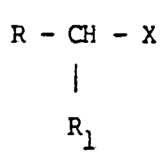

(V)

dans laquelle R et $R_1$ ont la signification donnée ci-avant et X représente un atome d'halogène.

3. Procédé selon la revendication 2, caractérisé en ce que dans les formules (I) et (V) $R_1$ représente

un atome d'hydrogène ou un radical alkyle inférieur.

4. Procédé selon l'une quelconque des revendications 1 à 3, caractérisé en ce que R représente un radical arylalkyle de formule générale :

$$(R')_n—Ar—(CH_2)_m—$$

dans laquelle : Ar représente un radical aryle comportant un ou deux cycles benzéniques condensés ou non ; R' représente un radical alkyle comportant de 1 à 4 atomes de carbone, un radical alkoxy comportant de 1 à 4 atomes de carbone, un atome d'halogène, un groupe nitrile, nitro, alkylcarbonyloxy ; n représente un nombre entier de 0 à 3 quand Ar comporte un seul cycle benzénique ou de 0 à 5 quand Ar comporte deux cycles benzéniques ; m représente un nombre entier de 1 à 20.

5. Procédé selon l'une quelconque des revendications 1 à 3, caractérisé en ce que dans les formules (I) et (V) R représente un radical aryloxyalkyle de formule générale :

$$(R')_n—Ar—O—(CH_2)_m—$$

ou un radical aryloxy de formule générale :

$$(R')_n—Ar—O—$$

dans lesquelles R', Ar, n et m ont la signification donnée à la revendication 4.

6. Procédé selon l'une quelconque des revendications 1 à 5, caractérisé en ce que le composé donneur d'hydrogène est pris dans le groupe formé par les alcools primaires et secondaires.

7. Procédé selon la revendication 6, caractérisé en ce que le donneur d'hydrogène est l'isopropanol.

8. Procédé selon l'une quelconque des revendications 1 à 7, caractérisé en ce que la quantité de donneur d'hydrogène est au moins voisine d'une mole par mole d'halogénure d'alkyle.

9. Procédé selon l'une quelconque des revendications 1 à 8, caractérisé en ce que la base minérale est un hydroxyde alcalin ou alcalino-terreux.

10. Procédé selon la revendication 9, caractérisé en ce que la base minérale est la chaux.

11. Procédé selon l'une quelconque des revendications 1 à 10, caractérisé en ce que la quantité de base est voisine d'une mole par mole d'halogénure d'alkyle.

12. Procédé selon l'une quelconque des revendications 1 à 11, caractérisé en ce que l'on utilise comme catalyseur un dérivé carbonyle du fer, du cobalt ou du nickel.

13. Procédé selon la revendication 12, caractérisé en ce que le catalyseur est pris dans le groupe formé par le dicobaltoctacarbonyle et les tétracarbonylcobaltates(-1) alcalins ou alcalino-terreux.

14. Procédé selon l'une quelconque des revendications 1 à 13, caractérisé en ce que la quantité de catalyseur exprimée en atomes-grammes de métal par mole d'halogénure d'alkyle est comprise dans un intervalle de 0,0001 à 1.

15. Procédé selon l'une quelconque des revendications 1 à 14, caractérisé en ce que la réaction est conduite en présence d'un tiers solvant inerte dans les conditions de la réaction.

16. Procédé selon la revendication 15, caractérisé en ce que la réaction est conduite dans l'eau ou solvant organique pris dans le groupe formé par les alcools tertiaires et les éthers et leur mélange avec l'eau.

17. Procédé selon la revendication 16, caractérisé en ce que le solvant est le t-butanol.

18. Procédé selon l'une quelconque des revendications 1 à 17, caractérisé en ce que la température à laquelle est conduite la réaction est comprise dans un intervalle de 90 à 160 °C.

19. Procédé selon l'une quelconque des revendications 1 à 18, caractérisé en ce que la pression d'oxyde de carbone à laquelle est conduite la réaction est comprise dans un intervalle de 1 à 200 bar.

## Claims

1. Process for preparing alpha-hydroxycarboxylic acids, characterized in that an alkyl halide is reacted with carbon monoxide and a hydrogen donor compound, in the presence of a carbonylation catalyst and an inorganic base, at a temperature above or equal to 90 °C.

2. Process according to claim 1, characterized in that alpha-hydroxycarboxylic acids of general formula :

$$R - \underset{\substack{|\\ R_1}}{CH} - \underset{\substack{|\\ OH}}{CH} - COOH \qquad (I)$$

in which R and $R_1$, which may be identical or different, denote :

a hydrogen atom ;

a linear or branched alkyl radical containing from 1 to 20 carbon atoms, optionally substituted by :

a) one or more functional residues which are inert under the conditions of the reaction, such as nitro, nitrile, alkylcarbonyloxy ;

b) one or more aryl residues ;

c) one or more cycloalkyl residues ;

d) one or more heterocyclic residues containing one or more hetero atoms chosen from the group consisting of oxygen, nitrogen and sulphur ;

e) an aryloxy radical ;

an aryloxy radical ;

are prepared from organic halides of general formula :

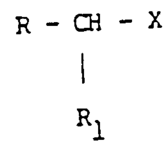

(V)

in which R and $R_1$ have the meaning given above and X denotes a halogen atom.

3. Process according to claim 2, characterized in that, in the formulae (I) and (V), $R_1$ denotes a hydrogen atom or a lower alkyl radical.

4. Process according to any one of claims 1 to 3, characterized in that R denotes an arylalkyl radical of general formula :

$$(R')_n—Ar—(CH_2)_m—$$

in which : Ar denotes an aryl radical containing one or two fused or unfused benzene rings ; R′ denotes an alkyl radical containing from 1 to 4 carbon atoms, an alkoxy radical containing from 1 to 4 carbon atoms, a halogen atom, a nitrile, nitro, alkylcarbonyloxy group ; n denotes an integer from 0 to 3 when Ar contains a single benzene ring or from 0 to 5 when Ar contains two benzene rings ; and m denotes an integer from 1 to 20.

5. Process according to any one of claims 1 to 3, characterized in that, in the formulae (I) and (C), R denotes an aryloxyalkyl radical of general formula :

$$(R')_n—Ar—O—(CH_2)_m—$$

or an aryloxy radical of general formula :

$$(R')_n—Ar—O—$$

in which R′, Ar, n and m have the meaning given in claim 4.

6. Process according to any one of claims 1 to 5, characterized in that the hydrogen donor compounds is chosen from the group consisting of primary and secondary alcohols.

7. Process according to claim 6, characterized in that the hydrogen donor is isopropanol.

8. Process according to any one of claims 1 to 7, characterized in that the amount of hydrogen donor is at least in the region of one mole per mole of alkyl halide.

9. Process according to any one of claims 1 to 8, characterized in that the inorganic base is an alkali metal hydroxide or alkaline earth metal hydroxide.

10. Process according to claim 9, characterized in that the inorganic base is lime.

11. Process according to any one of claims 1 to 10, characterized in that the amount of base is in the region of one mole per mole of alkyl halide.

12. Process according to any one of claims 1 to 11, characterized in that a carbonyl derivative of iron, cobalt or nickel is used as catalyst.

13. Process according to claim 12, characterized in that the catalyst is chosen from the group consisting of dicobalt octacarbonyl and alkali metal or alkaline earth metal tetracarbonylcobaltates (1-).

14. Process according to any one of claims 1 to 13, characterized in that the amount of catalyst, expressed as gram-atoms of metal per mole of alkyl halide, is within a range from 0.0001 to 1.

15. Process according to any one of claims 1 to 14, characterized in that the reaction is performed in the presence of a solubilizer which is inert under the conditions of the reaction.

16. Process according to claim 15, characterized in that the reaction is performed in water or an organic solvent chosen from the group consisting of tertiary alcohols and ethers, and mixtures thereof with water.

17. Process according to claim 16, characterized in that the solvent is t-butanol.

18. Process according to any one of claims 1 to 17, characterized in that the temperature at which the reaction is performed is within the range from 90 to 160 °C.

19. Process according to any one of claims 1 to 18, characterized in that the pressure of carbon monoxide at which the reaction is performed is within a range from 1 to 200 bars.

## Patentansprüche

1. Verfahren zur Herstellung von Alpha-Hydroxycarbonsäuren, dadurch gekennzeichnet, daß man ein Alkylhalogenid mit Kohlenmonoxid und einer Wasserstoff-Donor-Verbindung in Gegenwart eines Carbonylierungskatalysators und einer Mineralbase bei einer Temperatur von oberhalb oder gleich 90 °C reagieren läßt.

2. Verfahren nach Anspruch 1, dadurch gekennzeichnet, daß man Alpha-Hydroxycarbonsäuren der allgemeinen Formel :

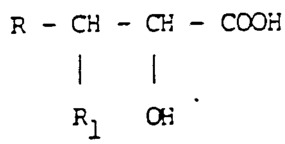

(I)

herstellt, in der R und $R_1$ identisch oder verschieden sind und jeweils darstellen :

ein Wasserstoffatom,

einen linearen oder verzweigten Alkylrest mit 1 bis 20 Kohlenstoffatomen, gegebenenfalls substituiert mit :

a) ein oder mehreren bei den Reaktionsbedingungen inerten funktionellen resten wie Nitro, Nitril, Alkylcarbonyloxy,

b) ein oder mehreren Arylresten,

c) ein oder mehreren Cycloalkylresten,

d) ein oder mehreren heterocyclischen Resten mit ein oder mehreren Heteroatomen ausgewählt aus der Gruppe gebildet aus Sauerstoff, Stickstoff und Schwefel,

e) einem Aryloxyrest ;

einen Aryloxyrest,

ausgehend von organischen Halogeniden der allgemeinen Formel :

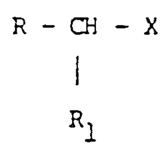

(V)

in der R und $R_1$ die oben angegebene Bedeutung haben und X ein Halogenatom darstellt.

3. Verfahren nach Anspruch 2, dadurch gekennzeichnet, daß in den Formeln (I) und (V) $R_1$ ein Wasserstoffatom darstellt oder einen niederen Alkylrest.

4. Verfahren nach einem der Ansprüche 1 bis 3, dadurch gekennzeichnet, daß R einen Arylalkylrest der allgemeinen Formel :

$$(R')_n—Ar—(CH_2)_m—$$

darstellt, in der : Ar einen Arylrest mit 1 oder 2 kondensierten oder nichtkondensierten Benzolresten darstellt ; R' einen Alkylrest mit 1 bis 4 Kohlenstoffatomen, einen Alkoxyrest mit 1 bis 4 Kohlenstoffatomen, ein Halogenatom, eine Nitril-, Nitro-oder Alkyl-carbonyloxygruppe darstellt ; n eine ganze Zahl von 0 bis 3 darstellt, wenn Ar einen einzigen Benzolring aufweist oder von 0 bis 5, wenn Ar zwei Benzolringe aufweist ; m eine ganze Zahl von 1 bis 20 darstellt.

5. Verfahren nach einem der Ansprüche 1 bis 3, dadurch gekennzeichnet, daß in den Formeln (I) und (V) R einen Aryloxyalkylrest der allgemeinen Formel :

$$(R')_n—Ar—O—(CH_2)_m—$$

darstellt oder einen Aryloxyrest der allgemeinen Formel :

$$(R')_n—Ar—O—$$

in der R', Ar, n und m die in Anspruch 4 angegebene Bedeutung haben.

6. Verfahren nach einem der Ansprüche 1 bis 5, dadurch gekennzeichnet, daß die Wasserstoff-Donor-Verbindung ausgewählt wird aus der Gruppe bestehend aus den primären und sekundären Alkoholen.

7. Verfahren nach Anspruch 6, dadurch gekennzeichnet, daß der Wasserstoff-Donor Isopropanol ist.

8. Verfahren nach einem der Ansprüche 1 bis 7, dadurch gekennzeichnet, daß die Menge an Wasserstoff-Donor wenigstens nahezu 1 Mol pro Mol Alkylhalogenid beträgt.

9. Verfahren nach einem der Ansprüche 1 bis 8, dadurch gekennzeichnet, daß die Mineralbase ein Alkalihydroxid oder Erdalkalihydroxid ist.

10. Verfahren nach Anspruch 9, dadurch gekennzeichnet, daß die Mineralbase Kalk ist.

11. Verfahren nach einem der Ansprüche 1 bis 10, dadurch gekennzeichnet, daß die Menge an Base in etwa 1 Mol pro Mol Alkylhalogenid beträgt.

12. Verfahren nach einem der Ansprüche 1 bis 11, dadurch gekennzeichnet, daß man als Katalysator ein Carbonylderivat des Eisens, des Kobalts oder des Nickels verwendet.

13. Verfahren nach Anspruch 12, dadurch gekennzeichnet, daß der Katalysator ausgewählt wird aus der Gruppe gebildet aus Dicobaltoctacarbonyl und den Alkali- oder Erdalkalitetracarbonylcobaltaten(-1).

14. Verfahren nach einem der Ansprüche 1 bis 13, dadurch gekennzeichnet, daß die Menge an Katalysator ausgedrückt in Grammatom Metall pro Mol Alkylhalogenid in einem Bereich von 0,0001 bis 1 liegt.

15. Verfahren nach einem der Ansprüche 1 bis 14, dadurch gekennzeichnet, daß die Reaktion in Gegenwart eines unter den Reaktionsbedingungen inerten Lösungvermittlers durchgeführt wird.

16. Verfahren nach Anspruch 15, dadurch gekennzeichnet, daß die Reaktion in Wasser oder einem organischen Lösungsmittel ausgewählt aus der Gruppe gebildet aus den tertiären Alkoholen und den Äthern sowie deren Gemisch mit Wasser durchgeführt wird.

17. Verfahren nach Anspruch 16, dadurch gekennzeichnet, daß das Lösungsmittel Tertiärbutanol ist.

18. Verfahren nach einem der Ansprüche 1 bis 17, dadurch gekennzeichnet, daß die Temperatur, bei der die Reaktion ausgeführt wird, in dem Bereich von 90 bis 160 °C liegt.

19. Verfahren nach einem der Ansprüche 1 bis 18, dadurch gekennzeichnet, daß der Druck an Kohlenmonoxid, bei dem die Reaktion durchgeführt wird ; in dem Bereich von 1 bis 200 bar liegt.